# EUROPEAN PATENT APPLICATION

(11) **EP 4 238 958 A1**
(43) Date of publication of application: **06.09.2023**
(21) Application number: 21922065.4
(22) Date of filing: 12.04.2021
(51) Int. Cl.: C07C 255/41, A01N 37/34, C07D 203/14, C07D 205/04, C07D 295/155, A01N 43/44, A01N 43/36, A01N 43/40, A01P 3/00

(54) **2-CYANOACRYLATE COMPOUND, PREPARATION METHOD THEREFOR AND USE THEREOF**

(30) Priority: 26.01.2021 CN 202110105207
(71) Applicant: Zhejiang University, Hangzhou, Zhejiang 310058 (CN); Zhejiang Research Institute of Chemical Industry Co., Ltd., Hangzhou, Zhejiang 310023 (CN); Jiangsu Pesticide Research Institute Co., Ltd, Nanjing, Jiangsu 210038 (CN)
(72) Inventor: MA, Zhonghua, Hangzhou city, Zhejiang 310058 (CN); WU, Ningjie, Hangzhou City, Zhejiang 310023 (CN); WANG, Honglei, Nanjing city, Jiangsu 210038 (CN); CHEN, Yun, Hangzhou city, Zhejiang 310058 (CN); XU, Tianming, Hangzhou City, Zhejiang 310023 (CN); CAO, Yang, Nanjing city, Jiangsu 210038 (CN)
(74) Representative: karo IP
(86) International application number: PCT/CN2021/086372
(87) International publication number: WO 2022/160466

(57) **Abstract**

The present disclosure discloses a 2-cyanoacrylate compound and use thereof in controlling a fungal disease of a crop, and belongs to the field of fungicides. The 2-cyanoacrylate compound has a structural formula shown in a formula (I), has excellent fungicidal activity, particularly has a very highly fungicidal activity on *Fusarium* species, and is suitable for controlling a fungal disease of a crop. Therefore, the compound may be used for preparing a fungicide in the fields of agriculture, horticulture, and the like, and is highly efficient, low in toxicity, and environmentally friendly.

## Description

### FIELD OF TECHNOLOGY

The present disclosure belongs to the field of fungicides and particularly relates to a 2-cyanoacrylate compound and use thereof in controlling a fungal disease of a crop.

### BACKGROUND

A 2-cyanoacrylate compound has pharmaceutical activities against tumors, and also has biological activities against weeding, pathogenic fungi, and plant viruses. Thus they are always valuable in the field of pesticide preparations. For example, a fungicide, phenamacril, may effectively prevent and treat diseases including wheat scab, rice bakanae disease, and the like.

Patent document CN1160318C discloses a class of 2-cyano-3-substituted phenyl acrylate compounds represented by the following general formula (a), which have a control effect on various diseases, such as wheat scab, caused by *Fusarium* spp.

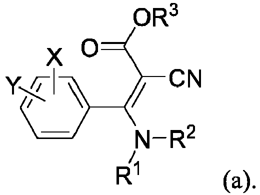

Patent document CN109879834A discloses a class of 2-cyano-3-aminoacrylate compounds represented by the following general formula (b). The mannich base fungicides have a control effect on plant diseases, such as wheat scab, caused by *Fusarium* spp.

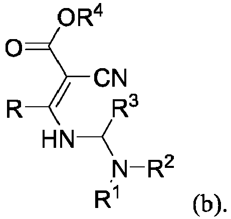

Patent document CN109867623A discloses a class of 3-pyridyl-3-amino-2-cyanoacrylate compounds represented by the following general formula (c), which have a control effect on various crop diseases, such as wheat scab, rice bakanae disease, watermelon Fusarium wilt, banana Fusarium wilt, and the like.

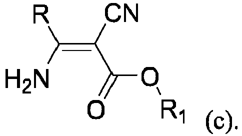

### SUMMARY

The present disclosure aims to provide a novel 2-cyanoacrylate compound with a high fungicidal activity, which is used for controlling a crop disease.

In order to realize the above objective, the present disclosure provides a 2-cyanoacrylate compound having a structural formula shown in a formula (I):

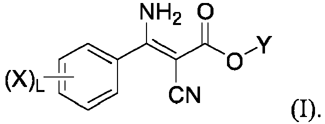

In the 2-cyanoacrylate compound shown in the general formula (I) provided by the present disclosure, L is an integer of 1-5.

As a preferred embodiment, the L is an integer of 1-4.

As a further preferred embodiment, the L is an integer of 1-3.

As a furthermore preferred embodiment, the L is an integer of 1-2.

In the 2-cyanoacrylate compound shown in the general formula (I) provided by the present disclosure, X is independently selected from -NR₁R₂, -N=CR₃R₄, -N=NR₅.

As a preferred embodiment, the X is selected from -NR₁R₂.

In the structure of the -NR₁R₂, R₁ and R₂ are independently selected from hydrogen, hydroxyl, amino, C₁-C₁₀ alkyl, halogenated C₁-C₁₀ alkyl, C₃-C₁₀ cycloalkyl, halogenated C₃-C₁₀ cycloalkyl, C₃-C₁₀ cycloalkyl C₁-C₆ alkyl, cyano C₁-C₁₀ alkyl, C₁-C₁₀ alkoxy, halogenated C₁-C₁₀ alkoxy, hydroxyl C₁-C₁₀ alkyl, C₁-C₁₀ alkoxy C₁-C₆ alkyl, halogenated C₁-C₁₀ alkoxy C₁-C₆ alkyl, C₂-C₁₀ alkenyl, C₂-C₁₀ alkenyl C₁-C₆ alkyl, C₂-C₁₀ alkynyl, C₂-C₁₀ alkynyl C₁-C₆ alkyl, C₁-C₁₀ alkylamino, C₁-C₁₀ dialkylamino, halogenated C₁-C₁₀ alkylamino, halogenated C₁-C₁₀ dialkylamino, amino C₁-C₁₀ alkyl, C₁-C₁₀ alkylamino C₁-C₆ alkyl, C₁-C₁₀ dialkylamino C₁-C₆ alkyl, C₁-C₁₀ alkylcarbonyl, C₁-C₁₀ alkoxycarbonyl, C₁-C₁₀ alkylaminocarbonyl, C₁-C₁₀ dialkylaminocarbonyl, C₁-C₁₀ alkylsulfonyl, C₁-C₁₀ alkoxysulfonyl, and C₁-C₁₀ alkylaminosulfonyl.

As a preferred embodiment, the substituents R₁ and R₂ are independently selected from hydrogen, hydroxyl, amino, C₁-C₆ alkyl, halogenated C₁-C₆ alkyl, C₃-C₆ cycloalkyl, halogenated C₃-C₆ cycloalkyl, C₃-C₆ cycloalkyl C₁-C₆ alkyl, cyano C₁-C₆ alkyl, C₁-C₆ alkoxy, halogenated C₁-C₆ alkoxy, hydroxyl C₁-C₆ alkyl, C₁-C₆ alkoxy C₁-C₆ alkyl, halogenated C₁-C₆ alkoxy C₁-C₆ alkyl, C₂-C₆ alkenyl, C₂-C₆ alkenyl C₁-C₆ alkyl, C₂-C₆ alkynyl, C₂-C₆ alkynyl C₁-C₆ alkyl, C₁-C₆ alkylamino, C₁-C₆ dialkylamino, halogenated C₁-C₆ alkylamino, halogenated C₁-C₆ dialkylamino, amino C₁-C₆ alkyl, C₁-C₆ alkylamino C₁-C₆ alkyl, C₁-C₆ dialkylamino C₁-C₆ alkyl, C₁-C₆ alkylcarbonyl, C₁-C₆ alkoxycarbonyl, C₁-C₆ alkylaminocarbonyl, C₁-C₆ dialkylaminocarbonyl, C₁-C₆ alkylsulfonyl, C₁-C₆ alkoxysulfonyl, and C₁-C₆ alkylaminosulfonyl.

As a further preferred embodiment, the substituents R₁ and R₂ are independently selected from hydrogen, hydroxyl, amino, C₁-C₃ alkyl, halogenated C₁-C₃ alkyl, C₃-C₆ cycloalkyl, halogenated C₃-C₆ cycloalkyl, C₃-C₆ cycloalkyl C₁-C₃ alkyl, cyano C₁-C₃ alkyl, C₁-C₃ alkoxy, halogenated C₁-C₃ alkoxy, hydroxyl C₁-C₃ alkyl, C₁-C₃ alkoxy C₁-C₃ alkyl, halogenated C₁-C₃ alkoxy C₁-C₃ alkyl, C₂-C₃ alkenyl, C₂-C₃ alkenyl C₁-C₃ alkyl, C₂-C₃ alkynyl, C₂-C₃ alkynyl C₁-C₃ alkyl, C₁-C₃ alkylamino, C₁-C₃ dialkylamino, halogenated C₁-C₃ alkylamino, halogenated C₁-C₃ dialkylamino, amino C₁-C₃ alkyl, C₁-C₃ alkylamino C₁-C₃ alkyl, C₁-C₃ dialkylamino C₁-C₃ alkyl, C₁-C₃ alkylcarbonyl, C₁-C₃ alkoxycarbonyl, C₁-C₃ alkylaminocarbonyl, C₁-C₃ dialkylaminocarbonyl, C₁-C₃ alkylsulfonyl, C₁-C₃ alkoxysulfonyl, and C₁-C₃ alkylaminosulfonyl.

As a furthermore preferred embodiment, the substituents R₁ and R₂ are independently selected from hydrogen, C₁-C₃ alkyl, halogenated C₁-C₃ alkyl, C₃-C₆ cycloalkyl, halogenated C₃-C₆ cycloalkyl, C₃-C₆ cycloalkyl C₁-C₃ alkyl, cyano C₁-C₃ alkyl, hydroxyl C₁-C₃ alkyl, C₁-C₃ alkoxy C₁-C₃ alkyl, halogenated C₁-C₃ alkoxy C₁-C₃ alkyl, amino C₁-C₃ alkyl, C₁-C₃ alkylamino C₁-C₃ alkyl, C₁-C₃ dialkylamino C₁-C₃ alkyl, C₁-C₃ alkylcarbonyl, C₁-C₃ alkoxycarbonyl, C₁-C₃ alkylaminocarbonyl, and C₁-C₃ dialkylaminocarbonyl.

In the structures of the -N=CR₃R₄ and the -N=NR₅, R₃, R₄, and R₅ are independently selected from hydrogen, hydroxyl, amino, C₁-C₁₀ alkyl, halogenated C₁-C₁₀ alkyl, C₃-C₁₀ cycloalkyl, halogenated C₃-C₁₀ cycloalkyl, C₁-C₁₀ alkoxy, halogenated C₁-C₁₀ alkoxy, C₁-C₁₀ alkylamino, C₁-C₁₀ dialkylamino, halogenated C₁-C₁₀ alkylamino, and halogenated C₁-C₁₀ dialkylamino.

As a preferred embodiment, the substituents R₃, R₄, and R₅ are independently selected from hydrogen, hydroxyl, amino, C₁-C₆ alkyl, halogenated C₁-C₆ alkyl, C₃-C₆ cycloalkyl, halogenated C₃-C₆ cycloalkyl, C₁-C₆ alkoxy, halogenated C₁-C₆ alkoxy, C₁-C₆ alkylamino, C₁-C₆ dialkylamino, halogenated C₁-C₆ alkylamino, and halogenated C₁-C₆ dialkylamino.

As a further preferred embodiment, the substituents R₃, R₄, and R₅ are independently selected from hydrogen, hydroxyl, amino, C₁-C₃ alkyl, halogenated C₁-C₃ alkyl, C₃-C₆ cycloalkyl, halogenated C₃-C₆ cycloalkyl, C₁-C₃ alkoxy, halogenated C₁-C₃ alkoxy, C₁-C₃ alkylamino, C₁-C₃ dialkylamino, halogenated C₁-C₃ alkylamino, and halogenated C₁₋₃ dialkylamino.

In the structure of the -NR₁R₂, the following cyclization forms may also exist: 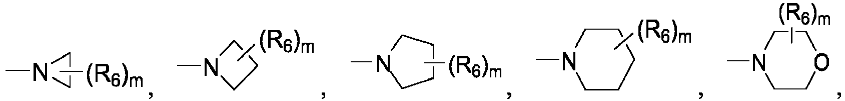 and

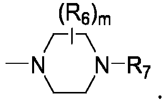

As a preferred embodiment, the structure of the -NR₁R₂ exists in the following cyclization forms:

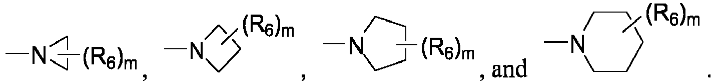

In the cyclization forms, R₆ and R₇ are independently selected from hydrogen, hydroxyl, amino, C₁-C₁₀ alkyl, halogenated C₁-C₁₀ alkyl, C₃-C₁₀ cycloalkyl, halogenated C₃-C₁₀ cycloalkyl, C₁-C₁₀ alkoxy, halogenated C₁-C₁₀ alkoxy, C₁-C₁₀ alkylamino, C₁-C₁₀ dialkylamino, halogenated C₁-C₁₀ alkylamino, and halogenated C₁-C₁₀ dialkylamino.

As a preferred embodiment, the substituents R₆ and R₇ are independently selected from hydrogen, hydroxyl, amino, C₁-C₆ alkyl, halogenated C₁-C₆ alkyl, C₃-C₆ cycloalkyl, halogenated C₃-C₆ cycloalkyl, C₁-C₆ alkoxy, halogenated C₁-C₆ alkoxy, C₁-C₆ alkylamino, C₁-C₆ dialkylamino, halogenated C₁-C₆ alkylamino, and halogenated C₁-C₆ dialkylamino.

As a further preferred embodiment, the substituents R₆ and R₇ are independently selected from hydrogen, hydroxyl, amino, C₁-C₃ alkyl, halogenated C₁-C₃ alkyl, C₃-C₆ cycloalkyl, halogenated C₃-C₆ cycloalkyl, C₁-C₃ alkoxy, halogenated C₁-C₃ alkoxy, C₁-C₃ alkylamino, C₁-C₃ dialkylamino, halogenated C₁-C₃ alkylamino, and halogenated C₁-C₃ dialkylamino.

As a furthermore preferred embodiment, the substituent R₆ is independently selected from hydrogen, C₁-C₃ alkyl, halogenated C₁-C₃ alkyl, C₃-C₆ cycloalkyl, and halogenated C₃-C₆ cycloalkyl.

In the cyclization forms, m is an integer of 1-4.

As a preferred embodiment, the m is an integer of 1-3.

As a further preferred embodiment, the m is an integer of 1-2.

In the 2-cyanoacrylate compound shown in the general formula (I) provided by the present disclosure, Y is independently selected from hydrogen, C₁-C₁₀ alkyl, halogenated C₁-C₁₀ alkyl, and C₃-C₁₀ cycloalkyl, or one of the following structures shown in Y₁ to Y₃:

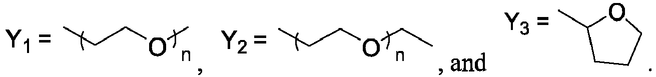

As a preferred embodiment, the substituent Y is independently selected from hydrogen, C₁-C₆ alkyl, halogenated C₁-C₆ alkyl, and C₃-C₆ cycloalkyl, or one of the following structures shown in Y₁ to Y₃.

As a further preferred embodiment, the substituent Y is independently selected from hydrogen, C₁-C₃ alkyl, halogenated C₁-C₃ alkyl, and C₃-C₆ cycloalkyl, or one of the following structures shown in Y₁ to Y₃.

As a furthermore preferred embodiment, the substituent Y is independently selected from hydrogen, C₁-C₃ alkyl, and halogenated C₁-C₃ alkyl, or one of the following structures shown in Y₁ to Y₂.

In the above structures shown in Y₁ to Y₃, n is an integer of 1-10.

As a preferred embodiment, the n is an integer of 1-6.

As a further preferred embodiment, the n is an integer of 1-3.

As a furthermore preferred embodiment, the L is an integer of 1-2.

Among the substituents of the present disclosure: alkyl refers to a straight or branched-chain form, such as methyl, ethyl, n-propyl, isopropyl, n-butyl, isobutyl, sec-butyl, tert-butyl, n-pentyl, isopentyl, n-hexyl, and the like; cycloalkyl refers to comprising a cyclic chain form, such as cyclopropyl, cyclobutyl, cyclopentyl, cyclohexyl, and the like; alkenyl means a straight or branched-chain form, such as vinyl, 1-propenyl, allyl, isopropenyl, 1-butenyl, 1,3-butadienyl, 1-hexenyl , and the like; alkynyl refers to a straight or branched-chain form, such as ethynyl, 1-propynyl, propargyl, 2-butynyl, 2-pentynyl, 3-hexynyl, and the like; halogenated alkyl refers to a group in which an alkyl is substituted with one or more halogen atoms; alkoxy refers to a group in which an oxygen atom attached to a terminal of an alkyl; halogenated alkoxy refers to a group in which an alkyl is substituted with one or more halogen atoms and an oxygen atom is attached to a terminal; halogen refers to fluorine, chlorine, bromine, and iodine; alkylamino refers to a group in which a nitrogen atom attached to a terminal of an alkyl; halogenated alkylamino refers to a group in which an alkyl is substituted with one or more halogen atoms and a nitrogen atom is attached to a terminal; alkylcarbonyl refers to a group in which a carbonyl is attached to a terminal of an alkyl; alkoxycarbonyl refers to a group in which an oxygen atom is attached to a terminal of an alkyl and the oxygen atom is then attached to a carbonyl; alkylaminocarbonyl refers to a group in which a terminal end of an alkyl is attached to a nitrogen atom and the nitrogen atom is attached to a carbonyl; alkylsulfonyl refers to a group in which a sulfonyl is attached to a terminal of an alkyl; alkoxysulfonyl refers to a group in which a nitrogen atom is attached to a terminal of an alkyl and the nitrogen atom is attached to a sulfonyl; and alkylaminosulfonyl refers to a group in which a nitrogen atom is attached to a terminal of an alkyl and the nitrogen atom is then attached to a carbonyl.

When X is -NR₁R₂ and L is 1, the 2-cyanoacrylate compound shown in the general formula (I) of the present disclosure has the following structural formula (I-1),

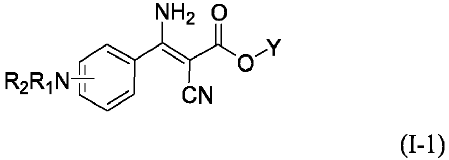

Index table 1 lists a typical compound represented by the structural formula (I-1), but the typical compound in the index table 1 does not limit the scope of the present disclosure.

**Index table 1**

| Compound No. | -NR₁R₂ | Y |
|---|---|---|
| 1 | 4-NH₂ | Et |
| 2 | 4-NH(Me) | Et |
| 3 | 4-NH(Et) | Et |
| 4 | 4-NH(n-Pr) | Et |
| 5 | 4-NH(n-Bu) | Et |
| 6 | 4-NH(i-Pr) | Et |
| 7 | 4-N(Me)₂ | Et |
| 8 | 4-N(Me)(Et) | Et |
| 9 | 4-N(Me)(n-Pr) | Et |
| 10 | 4-N(Me)(n-Bu) | Et |
| 11 | 4-N(Et)₂ | Et |
| 12 | 4-N(Et)(n-Pr) | Et |
| 13 | 4-N(Et)(n-Bu) | Et |
| 14 | 4-(aziridin-1-yl) | Et |
| 15 | 4-(azetidin-1-yl) | Et |
| 16 | 4-(pyrrolidin-1-yl) | Et |
| 17 | 4-(piperidin-1-yl) | Et |
| 18 | 4-morpholino | Et |
| 19 | 4-(piperazin-1-yl) | Et |
| 20 | 4-NH(acetyl) | Et |
| 21 | 4-N(Me)(acetyl) | Et |
| 22 | 4-NH(methylsulfonyl) | Et |
| 23 | 4-N(Me)(methylsulfonyl) | Et |
| 24 | 4-NH₂ | Me |
| 25 | 4-NH(Me) | Me |
| 26 | 4-NH(Et) | Me |
| 27 | 4-N(Me)₂ | Me |
| 28 | 4-N(Et)₂ | Me |
| 29 | 4-(aziridin-1-yl) | Me |
| 30 | 4-(azetidin-1-yl) | Me |
| 31 | 4-(pyrrolidin-1-yl) | Me |
| 32 | 4-(piperidin-1-yl) | Me |
| 33 | 4-NH₂ | n-Pr |
| 34 | 4-NH(Me) | n-Pr |
| 35 | 4-NH(Et) | n-Pr |
| 36 | 4-N(Me)₂ | n-Pr |
| 37 | 4-N(Et)₂ | n-Pr |
| 38 | 4-(aziridin-1-yl) | n-Pr |
| 39 | 4-(azetidin-1-yl) | n-Pr |
| 40 | 4-(pyrrolidin-1-yl) | n-Pr |
| 41 | 4-(piperidin-1-yl) | n-Pr |
| 42 | 4-NH₂ | i-Pr |
| 43 | 4-NH(Me) | i-Pr |
| 44 | 4-NH(Et) | i-Pr |
| 45 | 4-N(Me)₂ | i-Pr |
| 46 | 4-N(Et)₂ | i-Pr |
| 47 | 4-(aziridin-1-yl) | i-Pr |
| 48 | 4-(azetidin-1-yl) | i-Pr |
| 49 | 4-(pyrrolidin-1-yl) | i-Pr |
| 50 | 4-(piperidin-1-yl) | i-Pr |
| 51 | 4-NH₂ | n-Bu |
| 52 | 4-NH(Me) | n-Bu |
| 53 | 4-NH(Et) | n-Bu |
| 54 | 4-N(Me)₂ | n-Bu |
| 55 | 4-N(Et)₂ | n-Bu |
| 56 | 4-(aziridin-1-yl) | n-Bu |
| 57 | 4-(azetidin-1-yl) | n-Bu |
| 58 | 4-(pyrrolidin-1-yl) | n-Bu |
| 59 | 4-(piperidin-1-yl) | n-Bu |
| 60 | 4-NH₂ | CH₃CH₂OCH₂CH₂ |
| 61 | 4-NH(Me) | CH₃CH₂OCH₂CH₂ |
| 62 | 4-NH(Et) | CH₃CH₂OCH₂CH₂ |
| 63 | 4-N(Me)₂ | CH₃CH₂OCH₂CH₂ |
| 64 | 4-N(Et)₂ | CH₃CH₂OCH₂CH₂ |
| 65 | 4-(aziridin-1-yl) | CH₃CH₂OCH₂CH₂ |
| 66 | 4-(azetidin-1-yl) | CH₃CH₂OCH₂CH₂ |
| 67 | 4-(pyrrolidin-1-yl) | CH₃CH₂OCH₂CH₂ |
| 68 | 4-(piperidin-1-yl) | CH₃CH₂OCH₂CH₂ |
| 69 | 4-NH₂ | tetrahydrofuran-2-yl |
| 70 | 4-NH(Me) | tetrahydrofuran-2-yl |
| 71 | 4-NH(Et) | tetrahydrofuran-2-yl |
| 72 | 4-N(Me)₂ | tetrahydrofuran-2-yl |
| 73 | 4-N(Et)₂ | tetrahydrofuran-2-yl |
| 74 | 4-(aziridin-1-yl) | tetrahydrofuran-2-yl |
| 75 | 4-(azetidin-1-yl) | tetrahydrofuran-2-yl |
| 76 | 4-(pyrrolidin-1-yl) | tetrahydrofuran-2-yl |
| 77 | 4-(piperidin-1-yl) | tetrahydrofuran-2-yl |
| 78 | 3-NH₂ | Et |
| 79 | 3-NH(Me) | Et |
| 80 | 3-NH(Et) | Et |
| 81 | 3-NH(n-Pr) | Et |
| 82 | 3-NH(n-Bu) | Et |
| 83 | 3-NH(i-Pr) | Et |
| 84 | 3-N(Me)₂ | Et |
| 85 | 3-N(Me)(Et) | Et |
| 86 | 3-N(Me)(n-Pr) | Et |
| 87 | 3-N(Me)(n-Bu) | Et |
| 88 | 3-N(Et)₂ | Et |
| 89 | 3-N(Et)(n-Pr) | Et |
| 90 | 3-N(Et)(n-Bu) | Et |
| 91 | 3-(aziridin-1-yl) | Et |
| 92 | 3-(azetidin-1-yl) | Et |
| 93 | 3-(pyrrolidin-1-yl) | Et |
| 94 | 3-(piperidin-1-yl) | Et |
| 95 | 3-morpholino | Et |
| 96 | 3-(piperazin-1-yl) | Et |
| 97 | 3-NH(acetyl) | Et |
| 98 | 3-N(Me)(acetyl) | Et |
| 99 | 3-NH(methylsulfonyl) | Et |
| 100 | 3-N(Me)(methylsulfonyl) | Et |
| 101 | 3-NH₂ | Me |
| 102 | 3-NH(Me) | Me |
| 103 | 3-NH(Et) | Me |
| 104 | 3-N(Me)₂ | Me |
| 105 | 3-N(Et)₂ | Me |
| 106 | 3-(aziridin-1-yl) | Me |
| 107 | 3-(azetidin-1-yl) | Me |
| 108 | 3-(pyrrolidin-1-yl) | Me |
| 109 | 3-(piperidin-1-yl) | Me |
| 110 | 3-NH₂ | n-Pr |
| 111 | 3-NH(Me) | n-Pr |
| 112 | 3-NH(Et) | n-Pr |
| 113 | 3-N(Me)₂ | n-Pr |
| 114 | 3-N(Et)₂ | n-Pr |
| 115 | 3-(aziridin-1-yl) | n-Pr |
| 116 | 3-(azetidin-1-yl) | n-Pr |
| 117 | 3-(pyrrolidin-1-yl) | n-Pr |
| 118 | 3-(piperidin-1-yl) | n-Pr |
| 119 | 3-NH₂ | i-Pr |
| 120 | 3-NH(Me) | i-Pr |
| 121 | 3-NH(Et) | i-Pr |
| 122 | 3-N(Me)₂ | i-Pr |
| 123 | 3-N(Et)₂ | i-Pr |
| 124 | 3-(aziridin-1-yl) | i-Pr |
| 125 | 3-(azetidin-1-yl) | i-Pr |
| 126 | 3-(pyrrolidin-1-yl) | i-Pr |
| 127 | 3-(piperidin-1-yl) | i-Pr |
| 128 | 3-NH₂ | n-Bu |
| 129 | 3-NH(Me) | n-Bu |
| 130 | 3-NH(Et) | n-Bu |
| 131 | 3-N(Me)₂ | n-Bu |
| 132 | 3-N(Et)₂ | n-Bu |
| 133 | 3-(aziridin-1-yl) | n-Bu |
| 134 | 3-(azetidin-1-yl) | n-Bu |
| 135 | 3-(pyrrolidin-1-yl) | n-Bu |
| 136 | 3-(piperidin-1-yl) | n-Bu |
| 137 | 3-NH₂ | CH₃CH₂OCH₂CH₂ |
| 138 | 3-NH(Me) | CH₃CH₂OCH₂CH₂ |
| 139 | 3-NH(Et) | CH₃CH₂OCH₂CH₂ |
| 140 | 3-N(Me)₂ | CH₃CH₂OCH₂CH₂ |
| 141 | 3-N(Et)₂ | CH₃CH₂OCH₂CH₂ |
| 142 | 3-(aziridin-1-yl) | CH₃CH₂OCH₂CH₂ |
| 143 | 3-(azetidin-1-yl) | CH₃CH₂OCH₂CH₂ |
| 144 | 3-(pyrrolidin-1-yl) | CH₃CH₂OCH₂CH₂ |
| 145 | 3-(piperidin-1-yl) | CH₃CH₂OCH₂CH₂ |
| 146 | 3-NH₂ | tetrahydrofuran-2-yl |
| 147 | 3-NH(Me) | tetrahydrofuran-2-yl |
| 148 | 3-NH(Et) | tetrahydrofuran-2-yl |
| 149 | 3-N(Me)₂ | tetrahydrofuran-2-yl |
| 150 | 3-N(Et)₂ | tetrahydrofuran-2-yl |
| 151 | 3-(aziridin-1-yl) | tetrahydrofuran-2-yl |
| 152 | 3-(azetidin-1-yl) | tetrahydrofuran-2-yl |
| 153 | 3-(pyrrolidin-1-yl) | tetrahydrofuran-2-yl |
| 154 | 3-(piperidin-1-yl) | tetrahydrofuran-2-yl |

As a most preferable embodiment, the 2-cyanoacrylate compound shown in the general formula (I) is selected from at least one of the compounds shown in the following structural formulas,

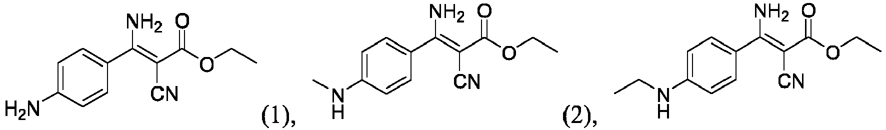

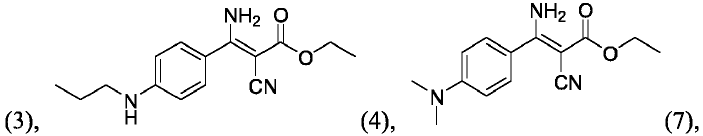

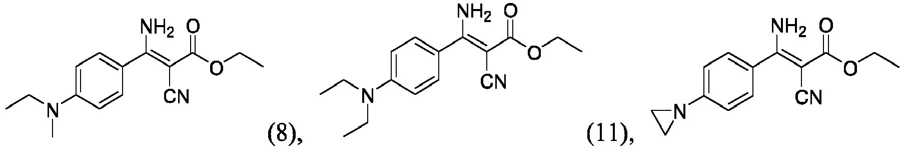

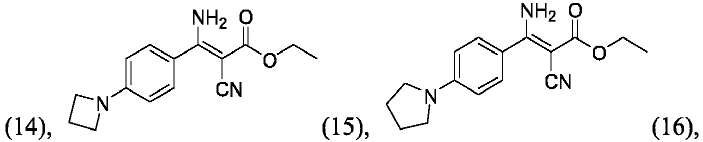

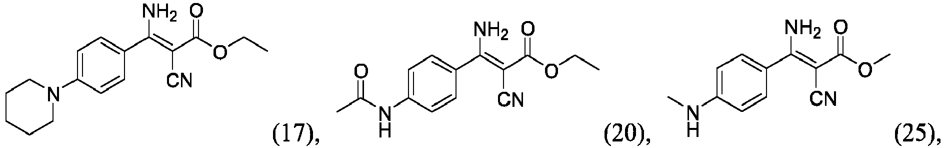

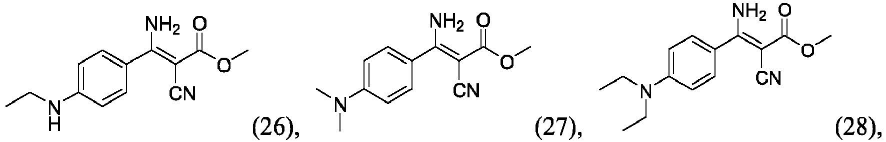

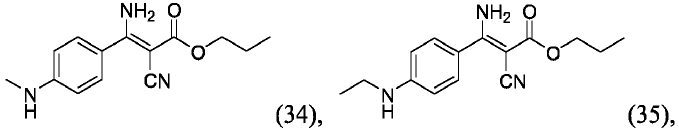

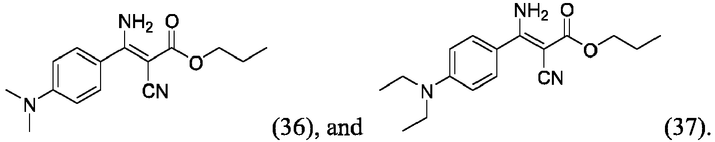

When X is -N=CR₃R₄ and L is 1, the 2-cyanoacrylate compound shown in the general formula (I) of the present disclosure has the following structural formula (I-2),

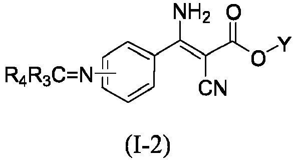

Index table 2 lists a typical compound represented by the structural formula (I-2), but the typical compound in the index table 2 does not limit the scope of the present disclosure.

**Index table 2**

| Compound No. | -N=CR₃R₄ | Y |
|---|---|---|
| 155 | 4-N=CH(Me) | Et |
| 156 | 4-N=CH(Et) | Et |
| 157 | 4-N=CH(n-Pr) | Et |
| 158 | 4-N=CH(i-Pr) | Et |
| 159 | 4-N=CH(n-Bu) | Et |
| 160 | 4-N=C(Me)₂ | Et |
| 161 | 4-N=C(Et)₂ | Et |
| 162 | 4-N=CH(OMe) | Et |
| 163 | 4-N=CH(NMe₂) | Et |
| 164 | 4-N=CH(Me) | Me |
| 165 | 4-N=CH(Et) | Me |
| 166 | 4-N=CH(n-Pr) | Me |
| 167 | 4-N=CH(i-Pr) | Me |
| 168 | 4-N=CH(n-Bu) | Me |
| 169 | 4-N=C(Me)₂ | Me |
| 170 | 4-N=C(Et)₂ | Me |
| 171 | 4-N=CH(OMe) | Me |
| 172 | 4-N=CH(NMe₂) | Me |
| 173 | 4-N=CH(Me) | n-Pr |
| 174 | 4-N=CH(Et) | n-Pr |
| 175 | 4-N=CH(n-Pr) | n-Pr |
| 176 | 4-N=CH(i-Pr) | n-Pr |
| 177 | 4-N=CH(n-Bu) | n-Pr |
| 178 | 4-N=C(Me)₂ | n-Pr |
| 179 | 4-N=C(Et)₂ | n-Pr |
| 180 | 4-N=CH(OMe) | n-Pr |
| 181 | 4-N=CH(NMe₂) | n-Pr |
| 182 | 4-N=CH(Me) | i-Pr |
| 183 | 4-N=CH(Et) | i-Pr |
| 184 | 4-N=CH(n-Pr) | i-Pr |
| 185 | 4-N=CH(i-Pr) | i-Pr |
| 186 | 4-N=CH(n-Bu) | i-Pr |
| 187 | 4-N=C(Me)₂ | i-Pr |
| 188 | 4-N=C(Et)₂ | i-Pr |
| 189 | 4-N=CH(OMe) | i-Pr |
| 190 | 4-N=CH(NMe₂) | i-Pr |
| 191 | 4-N=CH(Me) | n-Bu |
| 192 | 4-N=CH(Et) | n-Bu |
| 193 | 4-N=CH(n-Pr) | n-Bu |
| 194 | 4-N=CH(i-Pr) | n-Bu |
| 195 | 4-N=CH(n-Bu) | n-Bu |
| 196 | 4-N=C(Me)₂ | n-Bu |
| 197 | 4-N=C(Et)₂ | n-Bu |
| 198 | 4-N=CH(OMe) | n-Bu |
| 199 | 4-N=CH(NMe₂) | n-Bu |
| 200 | 3-N=CH(Me) | Et |
| 201 | 3-N=CH(Et) | Et |
| 202 | 3-N=CH(n-Pr) | Et |
| 203 | 3-N=CH(i-Pr) | Et |
| 204 | 3-N=CH(n-Bu) | Et |
| 205 | 3-N=C(Me)₂ | Et |
| 206 | 3-N=C(Et)₂ | Et |
| 207 | 3-N=CH(OMe) | Et |
| 208 | 3-N=CH(NMe₂) | Et |
| 209 | 3-N=CH(Me) | Me |
| 210 | 3-N=CH(Et) | Me |
| 211 | 3-N=CH(n-Pr) | Me |
| 212 | 3-N=CH(i-Pr) | Me |
| 213 | 3-N=CH(n-Bu) | Me |
| 214 | 3-N=C(Me)₂ | Me |
| 215 | 3-N=C(Et)₂ | Me |
| 216 | 3-N=CH(OMe) | Me |
| 217 | 3-N=CH(NMe₂) | Me |
| 218 | 3-N=CH(Me) | n-Pr |
| 219 | 3-N=CH(Et) | n-Pr |
| 220 | 3-N=CH(n-Pr) | n-Pr |
| 221 | 3-N=CH(i-Pr) | n-Pr |
| 222 | 3-N=CH(n-Bu) | n-Pr |
| 223 | 3-N=C(Me)₂ | n-Pr |
| 224 | 3-N=C(Et)₂ | n-Pr |
| 225 | 3-N=CH(OMe) | n-Pr |
| 226 | 3-N=CH(NMe₂) | n-Pr |
| 227 | 3-N=CH(Me) | i-Pr |
| 228 | 3-N=CH(Et) | i-Pr |
| 229 | 3-N=CH(n-Pr) | i-Pr |
| 230 | 3-N=CH(i-Pr) | i-Pr |
| 231 | 3-N=CH(n-Bu) | i-Pr |
| 232 | 3-N=C(Me)₂ | i-Pr |
| 233 | 3-N=C(Et)₂ | i-Pr |
| 234 | 3-N=CH(OMe) | i-Pr |
| 235 | 3-N=CH(NMe₂) | i-Pr |
| 236 | 3-N=CH(Me) | n-Bu |
| 237 | 3-N=CH(Et) | n-Bu |
| 238 | 3-N=CH(n-Pr) | n-Bu |
| 239 | 3-N=CH(i-Pr) | n-Bu |
| 240 | 3-N=CH(n-Bu) | n-Bu |
| 241 | 3-N=C(Me)₂ | n-Bu |
| 242 | 3-N=C(Et)₂ | n-Bu |
| 243 | 3-N=CH(OMe) | n-Bu |
| 244 | 3-N=CH(NMe₂) | n-Bu |

When X is -N=NR₅ and L is 1, the 2-cyanoacrylate compound shown in the general formula (I) of the present disclosure has the following structural formula (I-3),

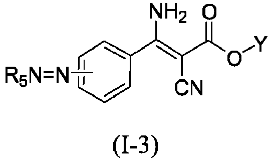

Index table 3 lists a typical compound represented by the structural formula (I-3), but the typical compound in the index table 3 does not limit the scope of the present disclosure.

**Index table 3**

| Compound No. | -N=NR₅ | Y |
|---|---|---|
| 245 | 4-N=N(Me) | Et |
| 246 | 4-N=N(Et) | Et |
| 247 | 4-N=N(n-Pr) | Et |
| 248 | 4-N=N(i-Pr) | Et |
| 249 | 4-N=N(n-Bu) | Et |
| 250 | 4-N=N(Me) | Me |
| 251 | 4-N=N(Et) | Me |
| 252 | 4-N=N(n-Pr) | Me |
| 253 | 4-N=N(i-Pr) | Me |
| 254 | 4-N=N(n-Bu) | Me |
| 255 | 4-N=N(Me) | n-Pr |
| 256 | 4-N=N(Et) | n-Pr |
| 257 | 4-N=N(n-Pr) | n-Pr |
| 258 | 4-N=N(i-Pr) | n-Pr |
| 259 | 4-N=N(n-Bu) | n-Pr |
| 260 | 4-N=N(Me) | i-Pr |
| 261 | 4-N=N(Et) | i-Pr |
| 262 | 4-N=N(n-Pr) | i-Pr |
| 263 | 4-N=N(i-Pr) | i-Pr |
| 264 | 4-N=N(n-Bu) | i-Pr |
| 265 | 4-N=N(Me) | n-Bu |
| 266 | 4-N=N(Et) | n-Bu |
| 267 | 4-N=N(n-Pr) | n-Bu |
| 268 | 4-N=N(i-Pr) | n-Bu |
| 269 | 4-N=N(n-Bu) | n-Bu |
| 270 | 3-N=N(Me) | Et |
| 271 | 3-N=N(Et) | Et |
| 272 | 3-N=N(n-Pr) | Et |
| 273 | 3-N=N(i-Pr) | Et |
| 274 | 3-N=N(n-Bu) | Et |
| 275 | 3-N=N(Me) | Me |
| 276 | 3-N=N(Et) | Me |
| 277 | 3-N=N(n-Pr) | Me |
| 278 | 3-N=N(i-Pr) | Me |
| 279 | 3-N=N(n-Bu) | Me |
| 280 | 3-N=N(Me) | n-Pr |
| 281 | 3-N=N(Et) | n-Pr |
| 282 | 3-N=N(n-Pr) | n-Pr |
| 283 | 3-N=N(i-Pr) | n-Pr |
| 284 | 3-N=N(n-Bu) | n-Pr |
| 285 | 3-N=N(Me) | i-Pr |
| 286 | 3-N=N(Et) | i-Pr |
| 287 | 3-N=N(n-Pr) | i-Pr |
| 288 | 3-N=N(i-Pr) | i-Pr |
| 289 | 3-N=N(n-Bu) | i-Pr |
| 290 | 3-N=N(Me) | n-Bu |
| 291 | 3-N=N(Et) | n-Bu |
| 292 | 3-N=N(n-Pr) | n-Bu |
| 293 | 3-N=N(i-Pr) | n-Bu |
| 294 | 3-N=N(n-Bu) | n-Bu |

The following table 4 is nuclear magnetic data of a part of compounds in the index tables 1-3. The compound number in table 4 corresponds to the compound number in index tables 1-3, i.e., the compound No. 1 in table 4 corresponds to the compound No. 1 described in index table 1. In table 4, s is a singlet, d is a doublet, dd is a doublet of doublets, t is a triplet, td is a triplet of doublets, q is a quartet, and m is a multiplet.

**Table 4. Nuclear magnetic data of compound**

| Compound No. | Nuclear magnetic analysis |
|---|---|
| 1 | ¹H NMR (400 MHz, CDCl₃) *δ*: 9.37 (s, 1H, -NH₂), 7.45 (d, *J* = 6.8 Hz, 2H, Ar-H), 6.70 (d, *J* = 6.8 Hz, 2H, Ar-H), 5.64 (s, 1H, -NH₂), 4.26 (q, *J* = 7.2 Hz, 2H, -CH₂), 1.35 (t, *J* = 7.2 Hz, 3H, -CH₃) ppm. |
| 2 | ¹H NMR (400 MHz, CDCl₃) *δ*: 9.37 (s, 1H, -NH₂), 7.48 (d, *J* = 8.0 Hz, 2H, Ar-H), 6.60 (d, *J* = 8.0 Hz, 2H, Ar-H), 5.66 (s, 1H, -NH₂), 4.26 (q, *J* = 7.2 Hz, 2H, -CH₂), 3.11 (s, 1H, -NH), 2.86 (s, 3H, -CH₃), 1.34 (t, *J* = 7.2 Hz, 3H, -CH₃) ppm. |
| 3 | ¹H NMR (400 MHz, CDCl₃) *δ*: 9.36 (s, 1H, -NH₂), 7.46 (d, *J* = 8.8 Hz, 2H, Ar-H), 6.58 (d, *J* = 8.8 Hz, 2H, Ar-H), 5.61 (s, 1H, -NH₂), 4.25 (q, *J* = 7.2 Hz, 2H, -CH₂), 4.06 (s, 1H, -NH), 3.18 (q, *J* = 7.2 Hz, 2H, -CH₂), 1.33 (t, *J* = 7.2 Hz, 3H, -CH₃), 1.26 (t, *J* = 7.2 Hz, 3H, -CH₃) ppm. |
| 7 | ¹H NMR (400 MHz, CDCl₃) *δ*: 9.34 (s, 1H, -NH₂), 7.53 (d, *J* = 8.8 Hz, 2H, Ar-H), 6.69 (d, *J* = 8.8 Hz, 2H, Ar-H), 5.74 (s, 1H, -NH₂), 4.25 (q, *J* = 7.2 Hz, 2H, -CH₂), 3.12 (s, 6H, -CH₃), 1.33 (t, *J* = 7.2 Hz, 3H, -CH₃) ppm. |
| 11 | ¹H NMR (400 MHz, CDCl₃) *δ*: 9.35 (s, 1H, -NH₂), 7.51 (d, *J* = 8.8 Hz, 2H, Ar-H), 6.65 (d, *J* = 8.8 Hz, 2H, Ar-H), 5.74 (s, 1H, -NH₂), 4.25 (q, *J* = 7.2 Hz, 2H, -CH₂), 3.38 (q, *J* = 7.2 Hz, 4H, -CH₂), 1.34 (t, *J* = 7.2 Hz, 3H, -CH₃), 1.18 (t, *J* = 7.2 Hz, 6H, -CH₃) ppm. |
| 20 | ¹H NMR (400 MHz, DMSO-d₆) *δ*: 10.19 (s, 1H, -NH), 9.17 (s, 1H, -NH₂), 8.82 (s, 1H, -NH₂), 7.65 (d, *J* = 7.2 Hz, 2H, Ar-H), 7.47 (d, *J* = 7.2 Hz, 2H, Ar-H), 4.12 (q, *J* = 7.2 Hz, 2H, -CH₂), 2.04 (s, 3H, -COCH₃), 1.19 (t, *J* = 7.2 Hz, 3H, -CH₃) ppm. |

The present disclosure further provides a preparation method for the 2-cyanoacrylate compound shown in the structural formula (I-1). The method comprises: 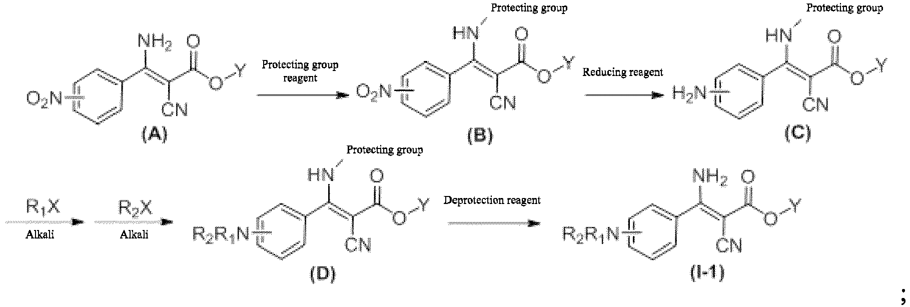 the substituents R₁, R₂, and Y are defined as before.

The preparation method comprises the following steps:
(1) reacting an intermediate (A) and a protecting group reagent to generate an intermediate (B) in an organic solvent at a temperature of 0-100°C, wherein the organic solvent is at least one selected from methanol, ethanol, toluene, dichloromethane, acetonitrile, acetone, tetrahydrofuran, dioxane, N,N-dimethylformamide, and dimethylsulfoxide; and the protecting group reagent is selected from at least one of an amino protecting group;
(2) reacting the intermediate (B) with a reducing reagent to generate an intermediate (C) in an organic solvent at a temperature of 0-100°C, wherein the organic solvent is at least one selected from methanol, ethanol, toluene, dichloromethane, acetonitrile, acetone, tetrahydrofuran, dioxane, N,N-dimethylformamide, and dimethylsulfoxide; and the reducing reagent is selected from at least one of common reducing reagents of iron powder, zinc powder, stannous chloride, hydrogen/palladium carbon, hydrogen/raney nickel, sodium hydrosulfite, hydrazine hydrate, and the like;
(3) reacting the intermediate (C) with R₁X and R₂X to generate an intermediate (D) through a two-step reaction in an organic solvent and in the presence of an alkali at a temperature of 0-100°C, wherein the organic solvent is at least one selected from methanol, ethanol, toluene, dichloromethane, acetonitrile, acetone, tetrahydrofuran, dioxane, N,N-dimethylformamide, and dimethylsulfoxide; the alkali is selected from at least one of an organic alkali and an inorganic alkali; and the R₁X and the R₂X are selected from at least one of an alkylation reagent, an acylation reagent, and a sulfonylation reagent; and
(4) reacting the intermediate (D) with a deprotection reagent to generate the 2-cyanoacrylate compound shown in a general formula (I-1) in an organic solvent at a temperature of 0-100°C, wherein the organic solvent is at least one selected from methanol, ethanol, toluene, dichloromethane, acetonitrile, acetone, tetrahydrofuran, dioxane, N,N-dimethylformamide, and dimethylsulfoxide; and the deprotection reagent is at least one of deprotection reagents aiming at an amino protecting group.

The present disclosure further provides use of the 2-cyanoacrylate compound shown in a general formula (I). The 2-cyanoacrylate compound shown in the general formula (I) is suitable for controlling a fungal disease.

When the 2-cyanoacrylate compound shown in the general formula (I) is used for controlling a fungal disease, the 2-cyanoacrylate compound shown in the general formula (I) is used for controlling a disease caused by at least one of *Fusarium, Bremia, Phytophthora, Alternaria, Gaeumannomyces, Ustilago, Aspergillus, Ascochyta, Botrytis,* and *Rhizoctonia.*

When the 2-cyanoacrylate compound shown in the general formula (I) is used for controlling a fungal disease, the 2-cyanoacrylate compound shown in the general formula (I) is used for controlling at least one disease of crop scab, bakanae disease, Fusarium wilt, rice blast, gray mold, and anthracnose.

The present disclosure further provides a pesticide formulation, wherein the pesticide formulation contains 0.001%-99.99% by weight of the 2-cyanoacrylate compound shown in the general formula (I). The pesticide formulation may be prepared into a missible oil, a suspending agent, a water suspending agent, a microemulsion, a (water) emulsion, a powder, a wettable powder, a soluble powder, a (water dispersible) granule or a capsule, and the like.

The present disclosure provides a pesticide formulation, wherein in addition to containing 0.001%-99.99% by weight of the 2-cyanoacrylate compound shown in the general formula (I), the pesticide formulation may further contain an agriculturally acceptable carrier.

The carrier may be a solid or a liquid. The suitable solid carrier comprises a natural or synthetic clay and silicate such as natural silica and diatomaceous earth; magnesium silicate such as talc; magnesium aluminum silicate such as kaolinite, kaolin, montmorillonite, and mica; white carbon black, calcium carbonate, and light calcium carbonate; calcium sulfate; limestone; sodium sulfate; and an amine salt such as ammonium sulfate and hexamethylene diamine. The liquid carrier comprises water and an organic solvent. When the water is used as a solvent or a diluent, the organic solvent may also be used as an adjuvant or an antifreeze additive. The suitable organic solvent comprises an aromatic hydrocarbon such as benzene, xylene, toluene, and the like; a chlorinated hydrocarbon such as chlorinated benzene, vinyl chloride, chloroform, dichloromethane, and the like; an aliphatic hydrocarbon such as a petroleum fraction, cyclohexane, and light mineral oil; an alcohol such as isopropyl alcohol, butyl alcohol, ethylene glycol, glycerin, cyclohexanol, and the like; an ether and an ester thereof; and also a ketone such as acetone, cyclohexanone, dimethylformamide, and N-methyl-pyrrolidone.

The carrier may also be a surfactant. The suitable surfactant may be an emulsifier, a disperser or a wetter, and may be ionic or non-ionic. The non-ionic emulsifier comprises a polyoxyethylene fatty acid ester, a polyoxyethylene fatty alcohol ether, a polyoxyethylene fatty amine, and commercially available emulsifiers: Nongru 2201B, Nongru 0203B, Nongru 100^{#}, Nongru 500^{#}, Nongru 600^{#}, Nongru 1601, Nongru 2201, Nongru NP-10, Nongru NP-15, Nongru 507^{#}, Nongru OX-635, Nongru OX-622, Nongru OX-653, Nongru OX-667, Ningru 36^{#}. The disperser comprises sodium lignosulfonate, nekal, calcium lignosulfonate, methyl naphthalene sulfonic acid formaldehyde condensate, and the like. The wetter comprises sodium laurylsulfate, sodium dodecylbenzenesulfonate, sodium alkylnaphthalenesulfonate, and the like.

The present disclosure further provides a sterilization method. The method comprises applying the 2-cyanoacrylate compound shown in the general formula (I) onto a pathogen needed to be controlled or a medium for growth thereof. The 2-cyanoacrylate compound shown in the general formula (I) is applied to onto the pathogen needed to be controlled or the medium for growth thereof at an amount of 10-1,000 g/hectare.

The present disclosure has the following beneficial effects:
The present disclosure provides a novel 2-cyanoacrylate compound. The compound has a highly fungicidal activity and particularly has a very high fungicidal activity against *Fusarium* fungi. Therefore, the compound may be used for preparing a fungicide in the fields of agriculture, horticulture, and the like, and is highly efficient, low in toxicity, and environmentally friendly.

### DESCRIPTION OF THE EMBODIMENTS

The present disclosure will be further illustrated in conjunction with specific examples, the present disclosure is not limited to these specific embodiments. It will be appreciated by those skilled in the art that the present disclosure covers all alternatives, modifications and equivalents as may be comprised within the scope of the claims.

In the present disclosure, unless otherwise specified, all the parts and percentages are in weight, all the used equipment, raw materials, and the like may be commercially available or commonly used in the industry. The methods in the examples are all conventional in the art, unless otherwise specified.

### I. Compound preparation

### Example 1 Synthesis of compound 3

### Step 1: synthesis of intermediate 3b

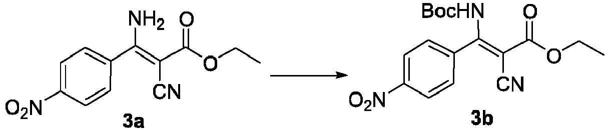

7.0 g of a raw material 3a and 150 mL of dichloromethane were added to a reaction flask, 11.7 g of di-tert-butyl dicarbonate was added in an ice bath, then 6.0 g of triethylamine and 3.3 g of DMAP were slowly added, and the materials were continuously stirred in an ice bath for 0.5 hour, heated to room temperature, and then stirred for 5 hours. After a reaction, 1 M hydrochloric acid was added to the reaction solution, and an organic phase was separated and then washed once each with pure water and a saturated salt solution. The organic phase was dried over anhydrous Na₂SO₄ and concentrated to obtain 9.6 g of an intermediate 3b, and the crude product was directly used in a next reaction without further purification.

### Step 2: synthesis of intermediate 3c

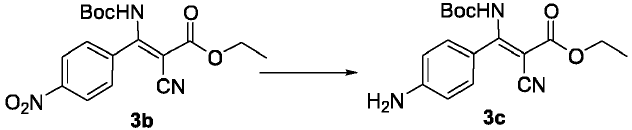

9.6 g of the intermediate 3b, 120 mL of ethanol, and 24 mL of water were added into a reaction flask, the reaction mixture was heated to 90°C. 3.7 g of iron powder and 12 mL of a saturated ammonium chloride solution were then added, and the reaction was continued for 4 hours. After the reaction was finished, the reaction mixture was filtered through diatomite. The filtrate was concentrated. Water was added and the mixture was extracted with ethyl acetate for three times. The organic phases were combined, and the combined organic phase was concentrated and separated by a column chromatography (an eluent was ethyl acetate and petroleum ether at a volume ratio of 1:2) to obtain 4.5 g of an intermediate 3c with a yield of 51.1%.

### Step 3: synthesis of intermediate 3d

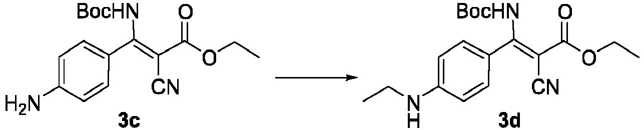

0.19 g of sodium hydride was added to 20 mL of tetrahydrofuran in an ice bath, then 1.0 g of the intermediate 3c was added and the materials were heated to room temperature and stirred for 30 minutes. Then 0.38 g of iodoethane was added, and the materials were stirred at room temperature overnight. After the reaction was finished, water was added to quench the reaction and the mixture was extracted with ethyl acetate for three times. The organic phases were combined, and the combined organic phase was concentrated and separated by a column chromatography (an eluent was ethyl acetate and petroleum ether at a volume ratio of 1:5) to obtain 0.52 g of an intermediate 3d with a yield of 48.1%.

### Step 4: synthesis of compound 3

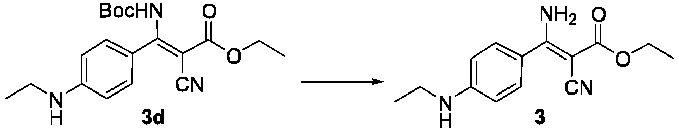

0.52 g of the intermediate 3d and 20 mL of tetrahydrofuran were added to a reaction bottle, the hydrogen chloride gas which was prepared by adding concentrated sulfuric acid into sodium chloride was slowly bubbled into a reaction solution for 1 hour. Then the reaction mixture was stirred at room temperature overnight. After the reaction, a saturated sodium bicarbonate aqueous solution was added and the mixture was extracted with ethyl acetate twice. The organic phases were combined, and the combined organic phase was concentrated and separated by a column chromatography (an eluent was ethyl acetate and petroleum ether at a volume ratio of 1:2) to obtain 0.35 g of a compound 3 with a yield of 93.3%.

### Example 2 Synthesis of compound 11

### Step 1: synthesis of intermediate 11d

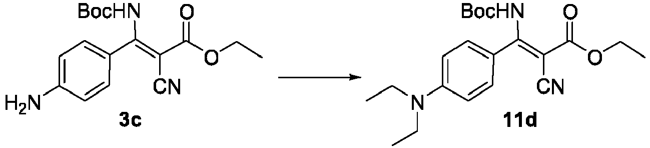

0.19 g of sodium hydride was added to 20 mL of tetrahydrofuran in an ice bath, then 1.0 g of the intermediate 3c was added, and the materials were heated to room temperature and stirred for 30 minutes. Then 2.07 g of iodoethane was added, and the materials were heated 60°C to react for 24 hours. After the reaction, water was added to quench the reaction and the mixture was extracted with ethyl acetate for three times. The organic phases were combined, and the combined organic phase was concentrated and separated by a column chromatography (an eluent was ethyl acetate and petroleum ether at a volume ratio of 1:2) to obtain 0.66 g of an intermediate 11d with a yield of 56.4%.

### Step 2: synthesis of compound 1

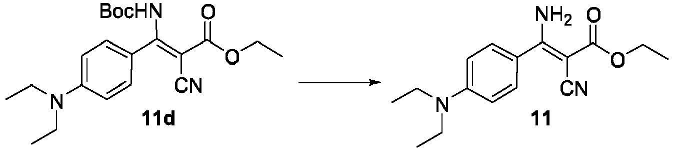

0.66 g of the intermediate 11d and 20 mL of dichloromethane were added to a reaction flask, 1.5 mL of trifluoroacetic acid was added dropwise under an ice bath, and the materials were stirred at room temperature overnight. After the reaction was finished, a saturated sodium bicarbonate aqueous solution was added and the mixture was extracted with ethyl acetate twice. The organic phases were combined, and the combined organic phase was concentrated and separated by a column chromatography (an eluent was ethyl acetate and petroleum ether at a volume ratio of 1:1) to obtain 0.45 g of a compound 11 with a yield of 91.8%.

### II. Formulation preparation

Practical examples of processing and preparing several fungicide formulations using the compound (I) of the present disclosure as an active substance are given in examples 3 to 7 below. It should be noted that the present disclosure is not limited to the scope of the following examples. In these formulation examples, all "%" refer to weight percentage.

### Example 3 Wettable powder formulation

15% of a compound (I) (index tables 1 and 2), 5% of a lignosulfonate (Mq), 1% of polyoxyethylene lauryl ether (JFC), 40% of diatomaceous earth, and 44% of light calcium carbonate were uniformly mixed and crushed to obtain a wettable powder.

### Example 4 Missible oil formulation

10% of a compound (I) (index tables 1 and 2), 5% of Nongru 500 (calcium salt), 5% of Nongru 602, 5% of N-methyl-2-pyrrolidone, and 75% of xylene were heated and stirred uniformly to obtain a missible oil.

### Example 5 Granule formulation

5% of a compound (I) (index tables 1 and 2), 1% of polyvinyl alcohol (PVA), 4% of a sodium naphthalenesulfonate formaldehyde condensate (NMO), and 90% of clay were mixed uniformly and crushed, 20 parts of water was added to 100 parts of the mixture, the materials were kneaded and prepared into a 14-32-mesh granule using an extruding granulator, and the granule was dried.

### Example 6 Water dispersible granule formulation

20% of a compound (I) (index tables 1 and 2), 4% of a naphthalenesulfonate formaldehyde condensate, 1% of a naphthalenesulfonate, 2% of white carbon black, and 73% of kaolin were mixed and crushed, then water was added, the materials were kneaded, and the kneaded materials were fed into a granulator equipped with a sieve having a certain specification for granulation. Then the granule was dried and sieved (according to a range of the sieve) to obtain a granular product.

### Example 7 Water suspension formulation

20% of a compound (I) (index tables 1 and 2), 1% of fatty alcohol-polyoxyethylene ether, 3% of rosin block polyoxyethylene ether polyoxypropylene ether sulfonate, 1% of magnesium aluminum silicate, 0.4% of an organic silicon defoamer, 5% of propylene glycol, and 69.5% of deionized water were mixed uniformly in advance, then the mixture was added into a sand grinder for sand grinding and filtered to obtain a suspension mother solution, a prepared xanthan gum (0.1%) water solution was added, and the materials were sheared and mixed uniformly.

### III. Activity test

An example of a biological activity assay using the compound of the present disclosure is given below. It should be noted that the present disclosure is not limited to the scope of the following example.

### Example 8 Antifungal activity measurement test

An inhibitory activity of the compound to be tested against a test pathogen was measured by using a mycelial growth rate method. The test pathogen was placed on a PDA plate. After growth rate of the pathogen reached a logarithmic phase, a fungal plug with a diameter of 0.5 cm was punched using a puncher on an edge of a fresh fungus colony and inoculated on the PDA plate containing a certain concentration of a drug to be tested. Meanwhile, the fungus plug inoculated on a PDA plate without a drug to be tested was used as a control. The inoculated PDA plates were cultured in a 25°C incubator for 2-3 days. When the test pathogen grew to the edge of the plate, the plates were examined. A colony growth diameter was measured using a cross method. A mycelial growth inhibition rate (MGIR) was calculated by the following formula: MGIR%=[(C-N)/C]×100%, wherein C is a colony diameter of a control group, and N is a colony diameter of a treated group. The experiment was repeated twice with 2 replicated dishes each time. The test pathogens included wheat scab fungi (*Fusarium graminearum* and *Fusarium asiaticum*), melon Fusarium wilt pathogen (*Fusarium oxysporum*), banana Fusarium wilt pathogen (*Fusarium oxysporum*), rice bakanae disease pathogen (*Fusarium moniliforme*), gray mold pathogen, and rice blast pathogen.

The present disclosure evaluated an *in-vitro* fungicidal activity of the numbered compounds in index tables 1-3. The result showed that: the compound of the present disclosure had highly fungicidal activity and particularly had excellent fungicidal activity against *Fusarium* fungi. "mg/L" refers to each mg of active matter per liter. Phenamacril was used as a control agent. The results were specifically as follows:

The compounds 1, 2, 3, 4, 7, 8, 11, 14, 15, 16, 17, 20, 25, 26, 27, 28, 34, 35, 36, and 37 at a concentration of 0.5 mg/L exhibited greater than 90% of MGIR against the scab pathogen, and the MGIR of phenamacril at the same concentration against the scab pathogen was about 70%. Consistently, under a concentration of 0.25 mg/L, the mycelial growth inhibition rates of compounds 2, 3, 4, 7, 8, 11, 14, 15, 16, and 17 against the scab pathogen were all greater than 90% and obviously higher than that of the control agent phenamacril. Moreover, under a concentration of 0.125 mg/L, the mycelial growth inhibition rates of compounds 2, 3, 7, and 11 against the scab pathogen were all greater than 90%, that are obviously higher than that of phenamacril. The mycelial growth inhibition rate of each compound was shown in the table below.

**Table 5**

| Compound No. | Mycelial growth inhibition rate of compound | | |
|---|---|---|---|
| | 0.5 mg/L | 0.25 mg/L | 0.125 mg/L |
| 1 | 90.2±4.3% | 81.2±6.3% | 65.5±4.3% |
| 2 | 99.6±1.3% | 95.2±1.8% | 90.6±7.6% |
| 3 | 99.8±3.3% | 95.4±7.7% | 91.6±2.8% |
| 4 | 96.5±5.5% | 90.5±2.7% | 84.3±3.8% |
| 7 | 99.9±3.0% | 96.8±5.7% | 94.8±6.1% |
| 8 | 98.5±2.5% | 91.7±3.3% | 88.3±4.5% |
| 11 | 99.9±3.0% | 95.3±7.1% | 92.2±3.4% |
| 14 | 96.5±5.6% | 90.7±1.3% | 85.3±3.3% |
| 15 | 95.5±2.5% | 90.5±3.3% | 84.2±4.5% |
| 16 | 95.2±4.5% | 90.2±4.4% | 82.3±2.5% |
| 17 | 94.2±4.5% | 90.3±5.7% | 81.4±5.5% |
| 20 | 90.1±1.9% | 79.8±1.5% | 60.2±2.3% |
| 25 | 92.3±3.3% | 85.1±4.3% | 75.3±6.8% |
| 26 | 92.5±4.0% | 84.6±2.3% | 77.2±5.5% |
| 27 | 91.2±4.3% | 84.2±2.9% | 76.7±7.0% |
| 28 | 92.8±5.0% | 85.3±3.3% | 74.4±3.8% |
| 34 | 92.2±6.3% | 84.9±5.4% | 74.1±2.3% |
| 35 | 93.4±4.3% | 85.8±1.8% | 76.6±3.3% |
| 36 | 93.5±4.8% | 85.4±3.9% | 75.2±3.3% |
| 37 | 92.2±5.4% | 84.8±7.1% | 73.9±5.5% |
| Phenamacril (control agent) | 69.7±7.7% | 36.1±5.0% | 21.1±2.3% |

In addition, compounds 2, 3, 7, and 11 also showed a good fungicidal activity on a part of a phenamacril-resistant scab pathogen. Under a concentration of 2 mg/L, the mycelial growth inhibition rates of compounds 2, 3, 7, and 11 on type-I myosin S217A, M375A, F419A, and I581A mutant strains were all greater than 60%, and the mycelial growth inhibition rates of the control agent phenamacril against these strains were only 1.2%, 25%, 19%, and 33% respectively.

Compounds 2, 3, 7, and 11 had a highly fungicidal activity against the melon Fusarium wilt pathogen, the banana Fusarium wilt pathogen, and the rice bakanae disease pathogen. Under a concentration of 1 mg/L, the mycelial growth inhibition rates of compounds 2, 3, 7, and 11 against these pathogens were all greater than 90%, and the mycelial growth inhibition rate of the control agent phenamacril on these pathogens were all 25%.

## Claims

1. A 2-cyanoacrylate compound, having a structural formula shown in a formula (I), 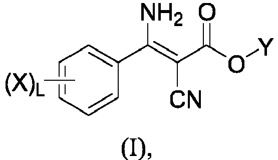
wherein X is independently selected from -NR₁R₂, -N=CR₃R₄, and -N=NR₅;
in the -NR₁R₂, R₁ and R₂ are independently selected from hydrogen, hydroxyl, amino, C₁-C₁₀ alkyl, halogenated C₁-C₁₀ alkyl, C₃-C₁₀ cycloalkyl, halogenated C₃-C₁₀ cycloalkyl, C₃-C₁₀ cycloalkyl C₁-C₆ alkyl, cyano C₁-C₁₀ alkyl, C₁-C₁₀ alkoxy, halogenated C₁-C₁₀ alkoxy, hydroxyl C₁-C₁₀ alkyl, C₁-C₁₀ alkoxy C₁-C₆ alkyl, halogenated C₁-C₁₀ alkoxy C₁-C₆ alkyl, C₂-C₁₀ alkenyl, C₂-C₁₀ alkenyl C₁-C₆ alkyl, C₂-C₁₀ alkynyl, C₂-C₁₀ alkynyl C₁-C₆ alkyl, C₁-C₁₀ alkylamino, C₁-C₁₀ dialkylamino, halogenated C₁-C₁₀ alkylamino, halogenated C₁-C₁₀ dialkylamino, amino C₁-C₁₀ alkyl, C₁-C₁₀ alkylamino C₁-C₆ alkyl, C₁-C₁₀ dialkylamino C₁-C₆ alkyl, C₁-C₁₀ alkylcarbonyl, C₁-C₁₀ alkoxycarbonyl, C₁-C₁₀ alkylaminocarbonyl, C₁-C₁₀ dialkylaminocarbonyl, C₁-C₁₀ alkylsulfonyl, C₁-C₁₀ alkoxysulfonyl, and C₁-C₁₀ alkylaminosulfonyl;
or the structure of the -NR₁R₂ is in a cyclization form: 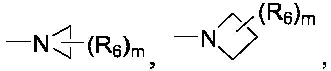 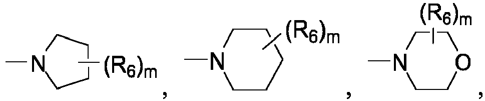 and 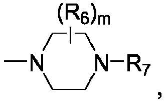
wherein R₆ and R₇ are independently selected from hydrogen, hydroxyl, amino, C₁-C₁₀ alkyl, halogenated C₁-C₁₀ alkyl, C₃-C₁₀ cycloalkyl, halogenated C₃-C₁₀ cycloalkyl, C₁-C₁₀ alkoxy, halogenated C₁-C₁₀ alkoxy, C₁-C₁₀ alkylamino, C₁-C₁₀ dialkylamino, halogenated C₁-C₁₀ alkylamino, and halogenated C₁-C₁₀ dialkylamino;
m is an integer of 1-4;
R₃, R₄, and R₅ are independently selected from hydrogen, hydroxyl, amino, C₁-C₁₀ alkyl, halogenated C₁-C₁₀ alkyl, C₃-C₁₀ cycloalkyl, halogenated C₃-C₁₀ cycloalkyl, C₁-C₁₀ alkoxy, halogenated C₁-C₁₀ alkoxy, C₁-C₁₀ alkylamino, C₁-C₁₀ dialkylamino, halogenated C₁-C₁₀ alkylamino, and halogenated C₁-C₁₀ dialkylamino;
L is an integer of 1-5; and
Y is independently selected from hydrogen, C₁-C₁₀ alkyl, halogenated C₁-C₁₀ alkyl, and C₃-C₁₀ cycloalkyl, or one of the following structures shown in Y₁ to Y₃: 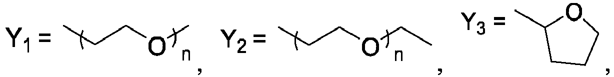
n is an integer of 1-10.

2. The 2-cyanoacrylate compound according to claim 1, wherein
in the -NR₁R₂, the R₁ and the R₂ are independently selected from hydrogen, hydroxyl, amino, C₁-C₆ alkyl, halogenated C₁-C₆ alkyl, C₃-C₆ cycloalkyl, halogenated C₃-C₆ cycloalkyl, C₃-C₆ cycloalkyl C₁-C₆ alkyl, cyano C₁-C₆ alkyl, C₁-C₆ alkoxy, halogenated C₁-C₆ alkoxy, hydroxyl C₁-C₆ alkyl, C₁-C₆ alkoxy C₁-C₆ alkyl, halogenated C₁-C₆ alkoxy C₁-C₆ alkyl, C₂-C₆ alkenyl, C₂-C₆ alkenyl C₁-C₆ alkyl, C₂-C₆ alkynyl, C₂-C₆ alkynyl C₁-C₆ alkyl, C₁-C₆ alkylamino, C₁-C₆ dialkylamino, halogenated C₁-C₆ alkylamino, halogenated C₁-C₆ dialkylamino, amino C₁-C₆ alkyl, C₁-C₆ alkylamino C₁-C₆ alkyl, C₁-C₆ dialkylamino C₁-C₆ alkyl, C₁-C₆ alkylcarbonyl, C₁-C₆ alkoxycarbonyl, C₁-C₆ alkylaminocarbonyl, C₁-C₆ dialkylaminocarbonyl, C₁-C₆ alkylsulfonyl, C₁-C₆ alkoxysulfonyl, and C₁-C₆ alkylaminosulfonyl;
or when the structure of the -NR₁R₂ is in a cyclization form, the R₆ and the R₇ are independently selected from hydrogen, hydroxyl, amino, C₁-C₆ alkyl, halogenated C₁-C₆ alkyl, C₃-C₆ cycloalkyl, halogenated C₃-C₆ cycloalkyl, C₁-C₆ alkoxy, halogenated C₁-C₆ alkoxy, C₁-C₆ alkylamino, C₁-C₆ dialkylamino, halogenated C₁-C₆ alkylamino, and halogenated C₁-C₆ dialkylamino; the m is an integer of 1-3;
the R₃, the R₄, and the R₅ are independently selected from hydrogen, hydroxyl, amino, C₁-C₆ alkyl, halogenated C₁-C₆ alkyl, C₃-C₆ cycloalkyl, halogenated C₃-C₆ cycloalkyl, C₁-C₆ alkoxy, halogenated C₁-C₆ alkoxy, C₁-C₆ alkylamino, C₁-C₆ dialkylamino, halogenated C₁-C₆ alkylamino, and halogenated C₁-C₆ dialkylamino;
the L is an integer of 1-4; and
the Y is independently selected from hydrogen, C₁-C₆ alkyl, halogenated C₁-C₆ alkyl, and C₃-C₆ cycloalkyl, or one of structures shown in Y₁ to Y₃, wherein n is an integer of 1-6.

3. The 2-cyanoacrylate compound according to claim 2, wherein
in the -NR₁R₂, the R₁ and the R₂ are independently selected from hydrogen, hydroxyl, amino, C₁-C₃ alkyl, halogenated C₁-C₃ alkyl, C₃-C₆ cycloalkyl, halogenated C₃-C₆ cycloalkyl, C₃-C₆ cycloalkyl C₁-C₃ alkyl, cyano C₁-C₃ alkyl, C₁-C₃ alkoxy, halogenated C₁-C₃ alkoxy, hydroxyl C₁-C₃ alkyl, C₁-C₃ alkoxy C₁-C₃ alkyl, halogenated C₁-C₃ alkoxy C₁-C₃ alkyl, C₂-C₃ alkenyl, C₂-C₃ alkenyl C₁-C₃ alkyl, C₂-C₃ alkynyl, C₂-C₃ alkynyl C₁-C₃ alkyl, C₁-C₃ alkylamino, C₁-C₃ dialkylamino, halogenated C₁-C₃ alkylamino, halogenated C₁-C₃ dialkylamino, amino C₁-C₃ alkyl, C₁-C₃ alkylamino C₁-C₃ alkyl, C₁-C₃ dialkylamino C₁-C₃ alkyl, C₁-C₃ alkylcarbonyl, C₁-C₃ alkoxycarbonyl, C₁-C₃ alkylaminocarbonyl, C₁-C₃ dialkylaminocarbonyl, C₁-C₃ alkylsulfonyl, C₁-C₃ alkoxysulfonyl, and C₁-C₃ alkylaminosulfonyl;
or when the structure of the -NR₁R₂ is in a cyclization form, the R₆ and the R₇ are independently selected from hydrogen, hydroxyl, amino, C₁-C₃ alkyl, halogenated C₁-C₃ alkyl, C₃-C₆ cycloalkyl, halogenated C₃-C₆ cycloalkyl, C₁-C₃ alkoxy, halogenated C₁-C₃ alkoxy, C₁-C₃ alkylamino, C₁-C₃ dialkylamino, halogenated C₁-C₃ alkylamino, and halogenated C₁-C₃ dialkylamino; the m is an integer of 1-2;
the R₃, the R₄, and the R₅ are independently selected from hydrogen, hydroxyl, amino, C₁-C₃ alkyl, halogenated C₁-C₃ alkyl, C₃-C₆ cycloalkyl, halogenated C₃-C₆ cycloalkyl, C₁-C₃ alkoxy, halogenated C₁-C₃ alkoxy, C₁-C₃ alkylamino, C₁-C₃ dialkylamino, halogenated C₁-C₃ alkylamino, and halogenated C₁₋₃ dialkylamino;
the L is an integer of 1-3; and
the Y is independently selected from hydrogen, C₁-C₃ alkyl, halogenated C₁-C₃ alkyl, and C₃-C₆ cycloalkyl, or one of structures shown in Y₁ to Y₃, wherein the n is an integer of 1-3.

4. The 2-cyanoacrylate compound according to claim 3, wherein
the X is independently selected from the -NR₁R₂;
the R₁ and the R₂ are independently selected from hydrogen, C₁-C₃ alkyl, halogenated C₁-C₃ alkyl, C₃-C₆ cycloalkyl, halogenated C₃-C₆ cycloalkyl, C₃-C₆ cycloalkyl C₁-C₃ alkyl, cyano C₁-C₃ alkyl, hydroxyl C₁-C₃ alkyl, C₁-C₃ alkoxy C₁-C₃ alkyl, halogenated C₁-C₃ alkoxy C₁-C₃ alkyl, amino C₁-C₃ alkyl, C₁-C₃ alkylamino C₁-C₃ alkyl, C₁-C₃ dialkylamino C₁-C₃ alkyl, C₁-C₃ alkylcarbonyl, C₁-C₃ alkoxycarbonyl, C₁-C₃ alkylaminocarbonyl, and C₁-C₃ dialkylaminocarbonyl;
or the structure of the -NR₁R₂ is in a cyclization form: 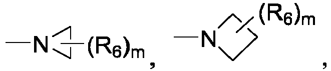 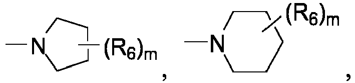 and
the R₆ is independently selected from hydrogen, C₁-C₃ alkyl, halogenated C₁-C₃ alkyl, C₃-C₆ cycloalkyl, and halogenated C₃-C₆ cycloalkyl; the m is an integer of 1-2;
the L is an integer of 1-2; and
the Y is independently selected from hydrogen, C₁-C₃ alkyl, and halogenated C₁-C₃ alkyl, or one of structures shown in Y₁ to Y₂, wherein the n is an integer of 1-2.

5. The 2-cyanoacrylate compound according to claim 4, wherein the compound is selected from at least one of the compounds shown in the following structural formulas,

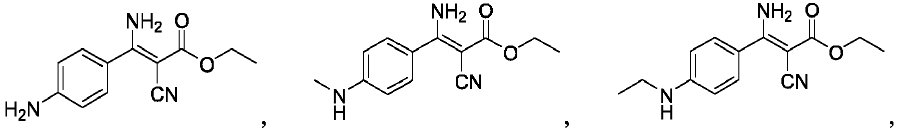

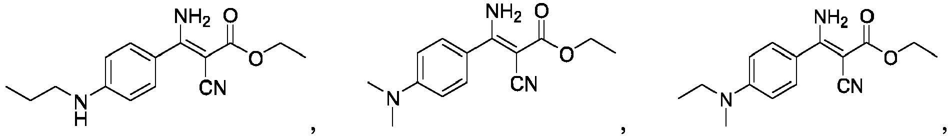

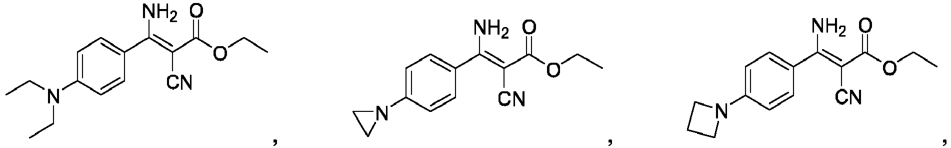

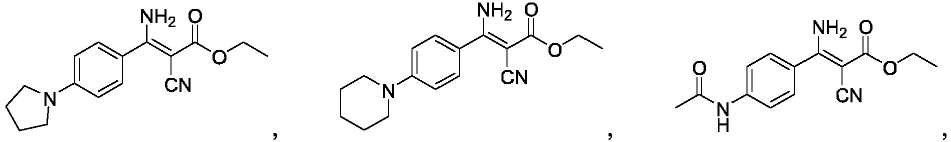

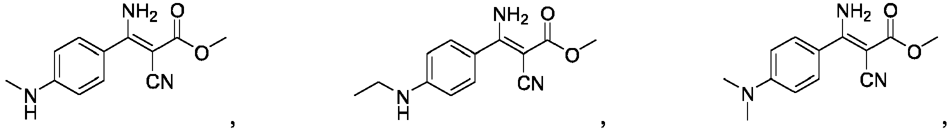

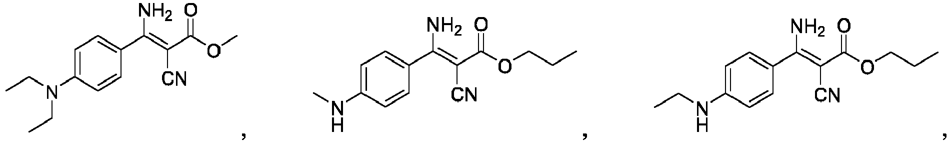

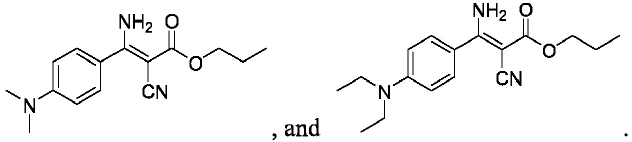

6. A preparation method for the 2-cyanoacrylate compound according to claim 1, wherein the X in a structure of the compound is -NR₁R₂, the L is 1, and the preparation method comprises the following steps:
(1) reacting a compound with a structural formula shown in (A) with an amino protecting group reagent to generate an intermediate B in an organic solvent at a temperature of 0-100°C; 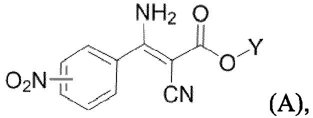
(2) reacting the intermediate B with a reducing reagent to generate an intermediate C in an organic solvent at a temperature of 0-100°C, wherein the reducing reagent is selected from at least one of iron powder, zinc powder, stannous chloride, hydrogen/palladium carbon, hydrogen/raney nickel, sodium hydrosulfite, and hydrazine hydrate;
(3) reacting the intermediate C with R₁X and R₂X to generate an intermediate D through a two-step reaction in an organic solvent and in the presence of an alkali at a temperature of 0-100°C, wherein the alkali is selected from at least one of an organic alkali and an inorganic alkali; and the R₁X and the R₂X are selected from at least one of an alkylation reagent, an acylation reagent, and a sulfonylation reagent; and
(4) reacting the intermediate D with a deprotection reagent to generate the 2-cyanoacrylate compound in an organic solvent at a temperature of 0-100°C, wherein the deprotection reagent is at least one of deprotection reagents aiming at an amino protecting group; and
the organic solvent in steps (1)-(4) is at least one selected from methanol, ethanol, toluene, dichloromethane, acetonitrile, acetone, tetrahydrofuran, dioxane, N,N-dimethylformamide, and dimethylsulfoxide.

7. Use of the 2-cyanoacrylate compound according to any one of claims 1-5 in controlling a fungal disease of a crop.

8. The use according to claim 7, wherein the fungal disease of a crop is a disease caused by at least one of *Fusarium, Bremia, Alternaria, Gaeumannomyces, Ustilago, Aspergillus, Ascochyta, Botrytis,* and *Rhizoctonia.*

9. The use according to claim 7, wherein the 2-cyanoacrylate compound is applied at an amount of 10-1,000 g/hectare.

10. A pesticide formulation, comprising 0.001%-99.99% by weight of the 2-cyanoacrylate compound according to any one of claims 1-5.
